# EUROPÄISCHE PATENTSCHRIFT

(11) **EP 0 943 098 B1**
(45) Veröffentlichungstag und Bekanntmachung des Hinweises auf die Patenterteilung: **09.03.2005**
(21) Anmeldenummer: 97952034.3
(22) Anmeldetag: 03.12.1997
(51) Int. Cl.: G01N 33/76, G01N 33/68, G01N 33/566

(54) **REZEPTORBINDUNGSASSAY ZUM NACHWEIS VON TSH-REZEPTOR-AUTOANTIKÖRPERN**
RECEPTOR BINDING ASSAY FOR DETECTING TSH-RECEPTOR AUTO-ANTIBODIES
ESSAI PAR FIXATION DES RECEPTEURS POUR DEPISTER DES AUTO-ANTICORPS DES RECEPTEURS DE TSH

(30) Priorität: 09.12.1996 DE 19651093
(43) Veröffentlichungstag der Anmeldung: 22.09.1999
(73) Patentinhaber: B.R.A.H.M.S Aktiengesellschaft, 16761 Hennigsdorf (DE)
(72) Erfinder: BERGMANN, Andreas, D-12351 Berlin (DE); STRUCK, Joachim, D-12161 Berlin (DE)
(74) Vertreter: Andrae, Steffen, Dr.
(86) Internationale Anmeldenummer: PCT/EP1997/006767
(87) Internationale Veröffentlichungsnummer: WO 1998/026294

(56) Entgegenhaltungen:
- DE-C- 4 120 412
- DE-C- 4 328 070
- DE-C- 19 522 171
- DE BRUIN T W A ET AL: "ANTI THYROTROPIN RECEPTOR ANTIBODIES IN GRAVES DISEASE AS DEMONSTRATED DIRECTLY BY IMMUNO PRECIPITATION ASSAY." ACTA ENDOCRINOL 102 (1). 1983. 49-56. CODEN: ACENA7 ISSN: 0001-5598, XP002061960
- YAVIN E ET AL: "MONO CLONAL ANTIBODIES TO THE THYROTROPIN RECEPTOR IMPLICATIONS FOR RECEPTOR STRUCTURE AND THE ACTION OF AUTO ANTIBODIES IN GRAVES DISEASE." PROC NATL ACAD SCI U S A 78 (5). 1981. 3180-3184. CODEN: PNASA6 ISSN: 0027-8424, XP002061961

## Beschreibung

Die vorliegende Erfindung betrifft einen kompetitiven Rezeptorbindungsassay zur Bestimmung von TSH-Rezeptor-Autoantikörpern, die bei Schilddrüsen-Autoimmunerkrankungen, insbesondere beim Morbus Basedow, auftreten.

Es ist bekannt, daß zahlreiche Erkrankungen, an denen die Schilddrüse beteiligt ist, Autoimmunerkrankungen sind, bei denen Autoantikörper gegen molekulare Strukturen der Schilddrüse gebildet werden, die im Zusammenhang mit der Erkrankung beginnen, als Autoantigene zu wirken. Die wichtigsten bekannten Auotantigene der Schilddrüse sind dabei Thyreoglobulin (Tg), die Schilddrüsenperoxidase (TPO) und insbesondere der TSH-Rezeptor (TSHr) (vgl. Furmaniak J et al., Autoimmunity 1990, Vol. 7, S. 63-80).

Der TSH-Rezeptor ist ein in der Schilddrüsenmembran lokalisierter Rezeptor, an den das von der Hypophyse ausgeschüttete Hormon TSH (Thyroid-stimulierendes Hormon oder Thyreotropin) bindet und dadurch die Ausschüttung der eigentlichen Schilddrüsenhormone, insbesondere des Thyroxins, auslöst. Der TSH-Rezeptor gehört zur Rezeptor-Familie der G-Protein-gekoppelten Glykoprotein-Rezeptoren mit einer großen aminoterminalen extrazellulären Domäne, zu der auch der LH/CG- und der FSH-Rezeptor gehören. Eine Aufklärung der chemischen Struktur des TSH-Rezeptors, d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur).

Es ist allgemein bekannt, daß bei der als Morbus Basedow (englisch: Graves' disease) bekannten Schilddrüsen-Autoimmunerkrankung stimulierende Autoantikörper eine Rolle spielen, die gegen der TSH-Rezeptor gebildet werden und mit diesem so wechselwirken, daß die Schilddrüse stimuliert wird, was sich als Schilddrüsenüberfunktion (Hyperthyreose) äußert. Die Bestimmung von Autoantikörpern gegen den TSH-Rezeptor hat somit für die Diagnose des Morbus Basedow eine erhebliche klinische Bedeutung.

Abgesehen von Bestimmungsverfahren, bei denen Versuchstiere oder spezielle Zellkulturen eine Rolle spielen und die heute vor allem von historischem Interesse sind (vgl. Schumm-Draeger et al., Akt. Endokr. Stoffw. 10 (1989), S. 90-102)), können TSH-Rezeptor-Autoantikörper bisher im wesentlichen nach zwei Verfahrensprinzipien bestimmt werden (vgl. Morgenthaler N.G. at al., Exp Clin Endocrinol Diabetes 104 (1996) Suppl 4, S.56-59) :

Bei Zellstimulationstests äußert sich die Anwesenheit von stimulierenden TSH-Rezeptor-Autoantikörpern, die in der Literatur häufig mit der Abkürzung TSI (TSI = thyroid stimulating immunoglobulins) bezeichnet werden, dadurch, daß bestimmte Funktionen von geeigneten Zellen, die in ihrer Zellmembran natürliche oder rekombinante TSH-Rezeptoren aufweisen und mit einer Autoantikörper enthaltenden Probe in Kontakt kommen, durch Stimulation ausgelöst oder verstärkt werden, insbesondere die Bildung von cAMP (cyclischem Adenosinmonophosphat). Bei diesen auch als Bioassays bezeichneten Tests wird selektiv die stimulierende Wirkung gemessen, die Messung ist jedoch außerordentlich aufwendig und daher für die klinische Routinediagnostik wenig geeignet.

Alternativ dazu können Autoantikörper auch unter Verwendung von kompetitiven Rezeptorbindungsassays, insbesondere Radiorezeptorassays, bestimmt werden, z.B. unter Verwendung des TRAK Assay® der B.R.A.H.M.S Diagnostica GmbH. Zur Bestimmung von TSH-Rezeptor-Autoantikörpern wird nach diesem herkömmlichen Verfahren so vorgegangen, daß man die zu bestimmenden Autoantikörper aus einer Serumprobe in flüssiger Phase mit einem radioaktiv markierten bovinen TSH-Kompetitor um die Bindungsstellen eines Detergens-solubilisierten porcinen TSH-Rezeptors konkurrieren läßt (vgl. Southgate, K. et al., Clin. Endocrinol. (Oxford) 20, 539-541 (1984); Matsuba T. et al., J.Biochem.118, S.265-270 (1995); EP 719 858 A2; Produktinformation zum TRAK-Assay® der Firma B.R.A.H.M.S Diagnostica GmbH). Um das an die Rezeptor-Präparation gebundene markierte TSH zu bestimmen, wird nach Abschluß der Inkubation der TSH-Rezeptor mit einem Fällungsreagens und einem anschließenden Zentrifugierschritt von der flüssigen Phase abgetrennt. Die Bestimmung des Rezeptor-gebundenen markierten TSH erfolgt durch Messung der im Sediment gebundenen Radioaktivität. Da die Bestimmung auf einer Konkurrenz (Kompetition) zwischen markiertem TSH und den zu bestimmenden Autoantikörpern um gemeinsame Bindungsstellen auf dem TSH-Rezeptor beruht, werden bei diesem Verfahren alle solchen und nur solche Autoantikörper erfaßt, die tatsächlich mit TSH kompetieren. Solche kompetierenden, zur Inhibierung der TSH-Bindung befähigten Autoantikörper werden in der Literatur auch als TBII (TBII = thyrotropin-binding inhibitory immunoglobulin) bezeichnet, und das Ausmaß ihrer Aktivität wird auch als prozentuale sogenannte TBII-Aktivität angegeben.

Es ist nunmehr seit längerem bekannt, daß bei Autoimmunerkrankungen der Schilddrüse heterogene Autoantikörperpopulationen unterschiedlicher Zusammensetzung gebildet werden. Dabei sind die stimulierenden und die mit TSH kompetierenden Autoantikörper nur teilweise identisch, d.h. es gibt stimulierende Autoantikörper, die nicht mit TSH kompetieren, und es gibt auch mit TSH kompetierende Autoantikörper, die nicht stimulierend wirken. Außerdem können auch noch Autoantikörper vorhanden sein, die weder stimulierend wirken, noch mit TSH kompetieren (vgl. z.B. Ludgate M et al., Mol. Cell. Endocrinol. 73 (1990), R13-R18; Filetti S et al., J. Clin. Endocrinol. Metab. 72; S.1096-1101, 1991; Morgenthaler N.G. at al., Exp Clin Endocrinol Diabetes 104 (1996) Suppl 4, S.56-59 und darin zitierte Literatur). Das hat zur Folge, daß nur bei etwa 80% bis 90% der Morbus Basedow Patienten mit Hilfe von Radiorezeptorassays Autoantikörper nachweisbar sind (vgl. z.B. Rationelle Diagnostik in der Endokrinologie, Thieme Verlag, S. 49, Absatz : TSH-Rezeptorautoantikörper (TSH-RAK); oder auch Ropars A et al., Cell. Immunol. 161, S.262-269 (1995); Ohmori M et al., Biochem. Biophys. Res. Commun. 174, No.1 (1991), S.399-403; Endo T. et al., Biochem. Biophys. Res. Commun. 181, No.3 (1991), S.1035-1041; Gupta MK, Clin. Biochem. 25, S. 193-199 (1992)). Da die Nichterfassung eines Teils der beim Morbus Basedow vorkommenden Autoantikörper ursächlich mit dem Meßprinzip der bisherigen kompetitiven Radiorezeptorassays verknüpft ist, wurde schon vorgeschlagen, trotz des erheblichen Aufwands eine ergänzende Bioassay-Messung zur Bestimmung stimulierender TSI Autoantikörper durchzuführen, wenn eine Diskrepanz zwischen dem klinische Bild eines Morbus Basedow Patienten und dem Meßergebnis der Bestimmung kompetierender TBII Autoantikörper erkennbar wird (Derwahl M et al., Exp. Clin. Endocrinol. 100 (1992), S. 75-79).

Außer der beschriebenen eingeschränkten klinischen Wertigkeit weisen die bisher bekannten kompetitiven Radiorezeptorassays zum Nachweis von TSH-Rezeptor-Autoantikörpern grundsätzliche Nachteile praktischer Natur auf, die darauf zurückzuführen sind, daß die Bindungsfähigkeit von TSH-Rezeptorpräparationen generell sehr empfindlich auf Veränderungen des Rezeptors oder des von ihm gebundenen Biomoleküls reagiert. Die Bindung von Biomolekülen von peptidischer oder proteinischer Natur, z.B. von Hormonen oder auch Autoantikörpern, an Rezeptoren ist in der Regel sehr komplexer Natur, und die Ausbildung einer spezifischen Bindung zwischen Rezeptor und Biomolekül ist sehr viel empfindlicher gegenüber strukturellen Veränderungen insbesondere des Rezeptors, als das bei einem üblichen Bindungspaar Antigen/Antikörper der Fall ist, das Grundlage der meisten Immunoassays ist, bei denen Rezeptoren keine Rolle spielen. Versuche, den TSH-Rezeptor zu immobilisieren und/oder zu markieren, führten in der Regel zu strukturellen Veränderungen, die die Funktionalität des Rezeptors stark beeinträchtigen. Das hat zur Folge, daß zahlreiche Assay-Grundtypen, die bei Immunoassays unter Ausnutzung einer Antikörper/Antigen-Bindung zur Verfügung stehen, insbesondere solche, bei denen mit immobilisierten Bindungspartnern gearbeitet wird und am Ende der Messung direkt die Konzentration einer an eine Festphase gebundenen Markierung bestimmt wird oder bei denen zur Markierung voluminöse Moleküle wie Enzyme, Enzymsubstrate oder Chemilumineszenzlabel verwendet werden, für die Durchführung von Rezeptorbindungsassays zur Bestimmung von TSH-Rezeptor-Autoantikörpern in der Praxis bisher nicht genutzt werden können. Da die Messung einer an eine Festphase gebundenen Markierung Grundlage der meisten Assay-Automaten für Reihenmessungen ist, sind die bekannten Assays zur Bestimmung von TSH-Rezeptor-Autoantikörpern bisher nicht auf derartigen Automaten durchführbar.

In dem Patent DE 43 28 070 C1 ist ein Typ eines Rezeptorbindungsassays, der nach der Coated-Tube-Technik arbeitet, beschrieben, bei dem die Schwierigkeit der Herstellung von markierten bzw. immobilisierten funktionalen Rezeptorpräparationen dadurch umgangen wird, daß man an die Festphase Bestandteile eines kompetitierenden Reaktionsystems bindet, das gewissermaßen einen "Schatten" der eigentlichen Rezeptorbindungsreaktion darstellt. Für die Schaffung von Assays für die klinische Routinediagnostik hat sich das offenbarte Verfahrensprinzip jedoch als zu kompliziert und daher wenig praktikabel erwiesen. Auf die allgemeinen Ausführungen in der genannten Patentschrift zur Problematik von Rezeptorbindungsassays im allgemeinen und von solchen zur Bestimmung von TSH-Rezeptor-Autoantikörpern im speziellen wird ergänzend ausdrücklich Bezug genommen.

Aus der EP-B-0 488 170 sind ferner zellfreie Rezeptorbindungstests bekannt, bei denen rekombinante Fusionsrezeptoren aus einem aminoterminalen Rezeptorprotein und einem Trägerprotein, insbesondere dem konstanten Teil (Fc) der schweren Kette eines Immunglobulins, eingesetzt werden, die mittels eines Antiserums oder eines monoklonalen Antikörpers an eine feste Phase gekoppelt sind. Die diskutierten Rezeptoren gehören nicht zur Klasse der hochmolekularen G-Protein gekoppelten Glykoprotein-Rezeptoren. Ferner ist eine Immobilisierung durch Bindung eines Trägerproteins, das der Fc-Teil eines Immunglobulins ist, für Rezeptorbindungsassays, mit deren Hilfe Autoantikörper bestimmt werden sollen, wenig geeignet, da die Autoantikörper selbst zu den Immunglobulinen gehören und an das Immobilisierungssystem binden können.

Aus der DE 41 20 412 C1 ist ferner ein Verfahren zur Bestimmung von Autoantikörpern bekannt, bei dem die Störung eines Sandwich, der aufgebaut wird aus einem immobilisierten spezifischen Antikörper, einem kruden Autoantigen in Form eines geeigneten Organextrakts, insbesondere TPO, und einem markierten weiteren Antikörper durch in der Probe vorhandene Autoantikörper gegen das Autoantigen zur Bestimmung genutzt wird. In einer Probe vorhandene Autoantikörper können im Sinne einer Störung prinzipiell mit jeder einzelnen oder auch beiden immunologischen Bindung(en) wechselwirken, die am Aufbau des vollständigen Sandwich beteiligt sind. Wegen der geschilderten Empfindlichkeit von Rezeptoren und dem Fehlen von Autoantikörpern mit der benötigten Selektivität war es bisher nicht möglich, dieses Verfahrensprinzip auf Fälle zu übertragen, bei denen das Autoantigen ein Rezeptor ist und der markierte Bindungspartner ein markiertes Hormon wie z.B. TSH.

Nachdem die Molekülstruktur des TSH-Rezeptors aufgeklärt war, wurden mit dem Ziel der Aufklärung der für die TSH-Bindung und die Antikörperbindung zuständigen Epitope des TSH-Rezeptors von zahlreichen Arbeitsgruppen monoklonale und polyklonale Antikörper gegen humane rekombinante TSH-Rezeptoren, gegen den (ohne das Signalpeptid 398 Aminosäuren umfassenden) N-terminalen extrazellulären Teil solcher Rezeptoren und gegen Konjugate kürzerer Rezeptorpeptid-Teilsequenzen hergestellt (vgl. z.B. N.G.Morgenthaler at al., Exp Clin Endocrinol Diabetes 104 (1996) Suppl 4, S.56-59; Seetharamaiah GS et al., Endocrinology 134, No. 2, S. 549-554 (1994); Desai RK et al., J. Clin. Endocrinol. Metab. 77:658-663, 1993; Dallas JS et al., Endocrinology 134, No. 3, S. 1437-1445 (1994); Johnstone AP et al., Mol. Cell. Endocrinol. 105 (1994), R1-R9; Seetharamaiah GS et al., Endocrinology 136, No. 7, S. 2817-2824 (1995); Nicholson LB et al., J. Mol. Endocrinol. (1996) 16, S. 159-170; Ropars A et al., Cell. Immunol. 161, S.262-269 (1995); Ohmori M et al., Biochem. Biophys. Res. Commun. 174, No.1 (1991), S.399-403; Endo T. et al., Biochem. Biophys. Res. Commun. 181, No.3 (1991), S.1035-1041; Costagliola S et al., Endocrinology 128, No.3, S.1555-1562, 1991; Marion S et al., Endocrinology 130, No.2, S.967-975 (1992); J. Sanders et al., J. Endocrinol. Invest. 19 (Suppl. No.6); 1996 und weitere, in den genannten Literaturstellen zitierte Veröffentlichungen). Die verschiedenen Antikörper wurden auf ihr Bindungsverhalten gegenüber dem TSH-Rezeptor bzw. rekombinant erzeugten Teilsequenzen davon und insbesondere auch im Hinblick auf ihre Fähigkeit, die Bindung von TSH an verschiedene Formen bzw. Fragmente des TSH-Rezeptors zu stören, geprüft. Da die verschiedenen polyklonalen oder monoklonalen Antikörper durch Immunisierung unterschiedlicher Tiere und/oder unter Verwendung von rekombinantem Material aus unterschiedlichen Expressionssystemen erzeugt worden waren und außerdem bei den Bindungstests häufig rekombinant erzeugte TSH-Rezeptoren unterschiedlichen Ursprungs bzw. Teilpeptide davon verwendet wurden, und da es sich ferner herausstellte, daß für die Bindung zahlreicher Antikörper die Glykolisierung und/oder korrekte Faltung der Rezeptorpeptide entscheidend sein dürfte, ist die Epitopstruktur von nativen TSH-Rezeptoren und das epitopspezifische Bindungsverhalten der in polyklonalen Autoantikörperpopulationen vorkommenden Autoantikörpern noch nicht umfassend geklärt.

In der Veröffentlichung Dallas JS et al., Endocrinology 134, No. 3, S. 1437-1445 (1994) wird beispielsweise ein mit Hilfe des Baculovirus/Insektenzellen-Expressionssystems hergestellter partieller rekombinanter TSH-Rezeptor, der die Aminosäuren der extrazellulären Domäne des humanen TSH-Rezeptors ohne das N-terminale Signalpeptid aufweist, dazu verwendet, Kaninchen zu immunisieren, und aus den gebildeten Immunglobulinfraktionen werden affinitätschromatographisch unter Verwendung synthetischer Peptide mit jeweils ca. 20 Aminosäuren spezifische Antikörperfraktionen gewonnen. Diese werden dann u.a. auf ihre Eignung untersucht, in einem kommerziellen Rezeptorbindungsassay die Bindung von TSH einen solubilisierten porcinen TSH-Rezeptor zu blockieren. Die Antikörper zeigten keine stimulatorische Aktivität.

In der Veröffentlichung Seetharamaiah GS et al., Endocrinology 136, No. 7, S. 2817-2824 (1995) wird beschrieben, wie der gleiche partielle rekombinante TSH-Rezeptor wie in der vorstehenden Veröffentlichung zur Immunisierung von Mäusen und zur Herstellung von monoklonalen Antikörpern gegen einzelne Epitope des TSH-Rezeptors nach Standardtechniken eingesetzt wurde. Eine ähnliche Vorgehensweise ist beschrieben in Nicholson LB et al., J. Mol. Endocrinol. (1996) 16, S. 159-170.

Gemäß Seetharamaiah GS et al., Endocrinology 134, No. 2, S. 549-554 (1994) wird ein wie vorstehend hergestellter partieller rekombinanter TSH-Rezeptor nachträglich gefaltet und nunmehr auf seine Eignung getestet, radioaktiv markiertes TSH zu binden. Dazu wird er in flüssiger Phase mit radioaktiv markiertem TSH umgesetzt. Um den entstanden Komplex möglichst quantitativ aus der Reaktionsmischung abzutrennen, wird dann als Teil eines Fällungssystems ein Antikörper zugesetzt, der durch Immunisierung von Kaninchen mit einem Konjugat eines Teilpeptids, das die Aminosäuren 357 bis 372 der vollständigen TSH-Rezeptorsequenz enthält, erzeugt wurde und von dem festgestellt worden war, daß er die Bindung von TSH an den ungefalteten partiellen rekombinanten TSH-Rezeptor nicht inhibierte (Desai RK et al., J. Clin. Endocrinol. Metab. 77:658-663, 1993). Der zugesetzte Antikörper bzw. die ihn und gebundenes radioaktiv markiertes TSH enthaltenden Komplexe werden dann mit Hilfe von Protein A, das unspezifisch an jegliche Antikörper bindet, ausgefällt. Unter den Versuchsbedingungen scheint die Bindung von Protein A an den rezeptorgebundenen Antikörper die gleichzeitige TSH-Bindung nicht zu beeinträchtigen.

Die mit Antikörpern gegen rekombinante TSH-Rezeptoren oder deren Teile gewonnenen Erkenntnisse führten zu dem Vorschlag, zur Bestimmung von TSH-Rezeptor-Autoantikörpern ein drittes, an sich bekanntes Verfahrensprinzip in Form eines Immunpräzipitationsassays zu nutzen, bei dem als Reagens zur Fällung eine Präparation eines extrazellulären Teil eines rekombinanten, durch Einbau von ³⁵S-markiertem Methionin markierten TSH-Rezeptors verwendet wird. Bei einem solchen Assay besteht weder eine Selektivität für TSI noch für TBII. (N.G.Morgenthaler at al., Exp Clin Endocrinol Diabetes 104 (1996) Suppl 4, S.56-59). Die Herstellung des markierten Rezeptors durch in vitro-Translation ist jedoch außerordentlich aufwendig und teuer, und es gibt keine Meßgeräte, die für eine klinische Routinemessung der von ³⁵S emittierten Strahlung geeignet sind. Das Verfahren ist daher als Meßverfahren für die klinische Routinediagnostik nicht geeignet.

Es ist Aufgabe der vorliegenden Erfindung, einen kompetitiven Rezeptorbindungsassay zur Bestimmung von TSH-Rezeptor-Autoantikörpern zu schaffen, der die beschriebenen Nachteile derartiger kompetitiver Rezeptorbindungsassays des Standes der Technik nicht aufweist.

Insbesondere ist es Aufgabe der vorliegenden Erfindung, einen verbesserten kompetitiven Rezeptorbindungsassay zur Bestimmung von TSH-Rezeptor-Autoantikörpern zu schaffen, bei dem es möglich ist, die aus den Reaktionspartnern des Bestimmungsverfahrens gebildeten TSH-Rezeptor-Komplexe zur Messung an einer üblichen Festphase zu immobilisieren und die bestehenden Einschränkungen bezüglich verwendbarer Markierungen zu überwinden, so daß auch eine automatisierte Durchführung derartiger Rezeptorbindungsassays möglich wird.

Insbesondere ist es ferner Aufgabe der vorliegenden Erfindung, derartige verbesserte kompetitive Rezeptorbindungsassays zum Nachweis von TSH-Rezeptor-Autoantikörpern so zu gestalten, daß gegenüber derartigen Assays des Standes der Technik eine erhöhte klinische Wertigkeit erhalten wird.

Es ist ferner Aufgabe der vorliegenden Erfindung, die zur Durchführung derartiger verbesserter Rezeptorbindungsassays in der klinischen Routinediagnostik erforderlichen Reagenziensätze (Kits) zu schaffen.

Die genannten Aufgaben werden bei einem kompetitiven Rezeptorbindungsassay gemäß Oberbegriff von Anspruch 1 wenigstens teilweise durch Radiorezeptorassays gelöst, die die im Kennzeichen des Anspruchs 1 wiedergegebenen Merkmale aufweisen.

Vorteilhafte Ausgestaltungen der erfindungsgemäßen verbesserten Rezeptorbindungsassays sind den Unteransprüchen zu entnehmen, insbesondere in Verbindung mit den nachfolgenden Erläuterungen in der vorliegenden Beschreibung.

Die Aufgabe der Schaffung eines entsprechenden Reagenziensatzes wird durch einen Reagenziensatz für die Durchführung eines erfindungsgemäßen Verfahrens gelöst, der wenigstens jeweils einen der Bestandteile (i), (ii) und (iii) gemäß Anspruch 15 enthält.

Um verschiedene mögliche Ausführungsformen für den Rezeptorbindungsassay der vorliegenden Erfindung abzudecken, wird in Anspruch 1 sowohl zur Beschreibung bekannter Merkmale derartiger Rezeptorbindungsassays als auch zur Charakterisierung solcher Merkmale, durch die sich ein erfindungsgemäßer Rezeptorbindungsassay von einem des Standes der Technik unterscheidet, eine verallgemeinernde Ausdrucksweise verwendet, die einleitend wie folgt ergänzend erläutert wird:

Wie im einleitenden Teil erläutert wurde, enthalten die bekannten kompetitiven Rezeptorbindungsassays, die alle als Radiorezeptorassays ausgestaltet sind, folgende Assaybestandteile:

Einen solubilisierten TSH-Rezeptor (i), insbesondere einen aus tierischen Schilddrüsenmembranen gewonnenen solubilisierten nativen tierischen (porcinen) TSH-Rezeptor.

Ferner enthalten die bekannten Radiorezeptorassays radioaktiv markiertes bovines TSH (ii), das mit TSH-Rezeptor-Autoantikörpern aus einer Serumprobe (TBII) um gemeinsame Bindungsstellen an dem verwendeten TSH-Rezeptor konkurriert. Da diese Kompetition diejenige ist, die bisher von primärer Bedeutung ist und auch eine Erfassung des überwiegenden Anteils auftretender Autoantikörper, nämlich 80 bis 90%, ermöglicht, fällt das markierte TSH des Standes der Technik unter die in Anspruch 1 verwendete Definition "primärer Kompetitor". Diese Definition umfaßt jedoch auch andere Formen von "primären Kompetitoren", deren Verwendung erstmals durch die vorliegende Erfindung ermöglicht wurde. So können als Kompetitoren für TBII oder gegebenenfalls auch andere Autoantikörperfraktionen auch markierte selektive Antikörper gegen den TSH-Rezeptor verwendet werden, wie sie nachfolgend näher beschrieben werden. Ferner soll, aufgrund der Kompetition mit TBII, auch ein festphasengebundenes TSH die Definition "primärer Kompetitor" erfüllen können.

Wie ebenfalls eingangs erläutert wurde, werden im Stand der Technik die Komplexe aus dem TSH-Rezeptor und daran gebundenen Bestandteilen der Reaktionsmischung, d.h. markiertem TSH und Autoantikörpern, mittels eines Fällungsreagenzes aus der Reaktionsmischung ausgefällt und durch Zentrifugieren davon abgetrennt. Im Sinne des Oberbegriffs von Anspruch 1 ist das Fällungsreagenz als Mittel gemäß (iii) anzusehen. Bei einem erfindungsgemäßen kompetitiven Rezept orbindungsassay entspricht einem derartigen Mittel (iii) ein festphasengebundener Umsetzungspartner, der in erster Linie ein an einer Festphase immobilisierter spezifischer TSH-Rezeptor-Antikörper ist, der jedoch gemäß einer "spiegelverkehrten" Ausführungsform auch ein festphasengebundenes TSH sein kann.

Es ist eine für die vorliegende Erfindung wesentliche Erkenntnis, daß es möglich ist, mit Hilfe immobilisierter selektiver Antikörper, die spezifisch gegen eine geeignete Teilsequenz des TSH-Rezeptors gerichtet sind, eine Immobilisierung auch eines nativen TSH-Rezeptors (d.h. eines humanen, tierischen oder auch rekombinanten TSH-Rezeptors, dessen Faltung/Glykosylierung einem natürlich vorkommenden TSH-Rezeptor wenigstens im wesentlichen entspricht) aus solubilisierten TSH-Rezeptor-Präparationen beliebiger Art zu erreichen, ohne daß die Bindungsfähigkeit des TSH-Rezeptors gegen TBII-Autoantikörper und markiertes TSH nennenswert beeinträchtigt ist. Mit dieser Erkenntnis wurde somit die Voraussetzung geschaffen, einen Rezeptorbindungsassay zur Bestimmung von TSH-Rezeptor-Autoantikörpern so durchzuführen, daß die Bestimmung der Menge des gebundenen markierten Kompetitors, z.B. des primären Kompetitors radioiodmarkiertes TSH, in festphasengebundener Form erfolgen kann. Überraschenderweise zeigte es sich jedoch ferner, daß es durch eine Immobilisierung des TSH-Rezeptors an eine Festphase mit Hilfe selektiver TSH-Rezeptor-Antikörper möglich ist, die klinische Wertigkeit von kompetitiven Rezeptorbindungsassays zur Bestimmung von TSH-Rezeptor-Autoantikörpern erheblich zu verbessern. Wie nachfolgend noch genauer erläutert wird, zeigte es sich nämlich, daß es bei einer geeigneten Wahl des an die Festphase gebundenen spezifischen Antikörpers dazu kommt, daß das Bestimmungssystem mindestens eine weitere Bindungsstelle enthält, um die Autoantikörper aus der Probe kompetieren können.

Wenn der festphasengebundene Antikörper so gewählt wird, daß er als "sekundärer Kompetitor" wirkt, der mit anderen Autoantikörpern der Probe als die durch Kompetition mit dem "primären Kompetitor" erfaßten TBII kompetiert, kommt es zu einer Erhöhung der klinischen Wertigkeit. Es wird nämlich ein Meßsystem gebildet, das als Analogie zu dem in DE 41 20 412 in Fig. 3 schematisch dargestellten Bestimmungssystem angesehen werden kann, bei dem es durch heterogene polyklonale Autoantikörper aus einer Patientenprobe zu einer doppelten Störung an unterschiedlichen Bindungsstellen eines Sandwich kommt. Da eine derartige Störung genügt, damit eine Bindung der Markierung an die Festphase verhindert wird, kommt es zu einer Erweiterung des Meßbereichs und einer Erhöhung der Empfindlichkeit. Die genannte Variante unter Verwendung zweier Antikörper und eines kruden Antigens wird auch von dem US-Patent 5,501,955 erfaßt.

Es zeigte sich nunmehr überraschend, daß nach dem erfindungsgemäßen Verfahren unter Verwendung von z.B. einem spezifischen Antikörper, der gegen ein synthetisches Peptid mit den Aminosäuren 20 bis 39 der Aminosäuresequenz des vollständigen TSH-Rezeptors (einschließlich Signalpeptid) erzeugt worden war, alle aufgrund klinischer Symptome als Morbus Basedow-Patienten eingestufte Patienten als Autoantikörper-positiv erfaßt wurden, obwohl mit dem herkömmlichen Radiorezeptorassay nur 80% als positiv ermittelt worden waren. Es ist somit durch Verwendung eines "sekundären Kompetitors", der mit anderen Autoantikörpern als TBII kompetiert, die bei Morbus Basedow auftreten, möglich, die klinische Wertigkeit des Rezeptorbindungsassays für die Diagnose des Morbus Basedow erheblich zu verbessern.

Der "sekundäre Kompetitor" kompetiert dabei vorzugsweise mit im Zellstimulationstest als stimulierend erfaßten Autoantikörpern (TSI-Autoantikörpern), die mangels Kompetition mit TSH durch den herkömmlichen Radiorezeptorassay nicht erfaßt werden. Es ist jedoch nicht erforderlich, daß die Autoantikörper, mit denen der "sekundäre Kompetitor" kompetiert, tatsächlich stimulierend wirken und damit die klinischen Symptome des Morbus Basedow verstärken. Die von dem "sekundären Kompetitor" kompetierten Autoantikörper können auch einer Autoantikörper-Subpopulation angehören, die blockierend wirkt oder für das eigentliche Krankheitsgeschehen unwichtig ist. Solange eine derartige Autoantikörper-Subpopulation stets oder häufig im Serum von Morbus Basedow-Patienten auftritt, wird die klinische Wertigkeit des Rezeptorbindungsassays gegenüber dem herkömmlichen Radiorezeptorassay erhöht, der nur TBII-Autoantikörper erfaßt.

Als selektive Antikörper, die bevorzugte "sekundäre Kompetitoren" bilden, kommen somit insbesondere solche in Frage, die gegen Abschnitte des TSH-Rezeptors gerichtet sind, die nicht an der TSH-Bindung beteiligt sind, gegen die jedoch beim Morbus Basedow Autoantikörper gebildet werden. Die bisherigen Untersuchungen unter Verwendung von affinitätschromatographisch gereinigten polyklonalen oder als monoklonale Antikörper hergestellten Antikörpern führten dazu, daß einige Abschnitte des TSH-Rezeptors als geeignete Bindungsregionen für derartige selektive Antikörper besonders in Betracht kommen. Bei der Interpretation der bisherigen Literaturdaten ist jedoch zu beachten, daß die meisten selektiven Antikörper gegen rekombinant erzeugte TSH-Rezeptorpeptide oder synthetische Teilpeptide erzeugt wurden und daher wahrscheinlich sequenzieller Natur sind. Ein Großteil der in der Natur beschriebenen Antikörper erwies sich ferner im Zellstimulationstest als wirkungslos. Das deutet darauf hin, daß trotz einer Kompetition mit Autoantikörpern aus Seren eine Gleichsetzung der künstlich erzeugten Antikörper mit den zu bestimmenden Autoantikörpern nicht möglich ist. Das Bindungsverhalten der künstlich erzeugten Antikörper an native TSH-Rezeptor-Präparationen kann sich ferner aufgrund der Faltung des nativen TSH-Rezeptors und seiner Glycosylierung vom Bindungsverhalten an synthetische Peptide oder rekombinant erzeugte TSH-Rezeptor-Fragmente unterscheiden. In Kenntnis der Lehre der vorliegenden Erfindung ist es jedoch im Einzelfall schon in relativ einfachen Routineversuchen möglich, die Eignung spezieller spezifischer Antikörper für das erfindungsgemäße Verfahren und ihre Fähigkeit, als "sekundärer Kompetitor" zu wirken, festzustellen.

Die nachfolgend beschriebenen Versuche wurden durchgeführt unter Verwendung eines immobilisierten spezifischen Antikörpers, von dem bekannt ist, daß er an ein Peptid aus dem Bereich der Aminosäuren 20 bis 39 des vollständigen TSH-Rezeptors bindet, sowie von markiertem bovinem TSH als herkömmlicher Tracer ("primärer Kompetitor"). Der verwendete Antikörper war ein monoklonaler Antikörper, der nach Immunisierung mit Hilfe eines Konjugats aus dem entsprechenden synthetischen Peptid nach der üblichen Hybridomtechnik selektiert und hergestellt worden war. In der Literatur wurden verschiedene polyklonale oder monoklonale spezifische Antikörper beschrieben, die ebenfalls an die gleiche Region oder überlappende Regionen des aminoterminalen Endes der extrazellulären Domäne des TSH-Rezeptors binden (vgl. Dallas J.S. et al., Endocrinology 134, No. 3, S. 1437-1445 (1994); Seetharamaiah G.S. et al., Endocrinology 136, No. 7, S. 2817-2824 (1995); Nicholson LB et al., J. Mol. Endocrinol. (1996) 16 S. 259-170; Ohmori, M. et al., J. Endocrinol. (1992) 135, S. 479-484; Endo, T. et al., Biochem. Biophys. Res. Commun. 177, S. 145-150 (1991); Hirooka, Y. et al., Med. Sci. Res. 1992, 20, S. 639-640). Die in den genannten Literaturstellen beschriebenen Herstellungstechniken und Antikörpertypen kommen grundsätzlich als Austausch für den in den Versuchen konkret verwendeten Antikörper in Frage, wobei jedoch zu beachten ist, daß bekannt ist, daß Antikörper gegen eng benachbarte Sequenzen des TSH-Rezeptors zu ganz unterschiedlichen Antikörpertypen gehören können.

Als Alternativen zu dem in den Beispielen verwendeten Antikörper, der gegen die Aminosäuren 20 bis 39 des humanen TSH-Rezeptors gerichtet ist, kommen auch Antikörper in Frage, die beispielsweise gegen die Regionen 32 bis 54, 287 bis 301, 361 bis 381 oder 739 bis 758 erzeugt wurden. Die speziellen Eigenschaften sind jedoch im Einzelfalle in Anlehnung an die nachfolgend beschriebenen Versuche zu prüfen.

Wenn man noch stärker von dem ursprünglichen Assaydesign des herkömmlichen Radiorezeptorassays abweicht und anstelle von radioaktiv markiertem TSH einen weiteren markierten selektiven Antikörper verwendet, kann die obige Unterscheidung von "primärem Kompetitor" und "sekundärem Kompetitor" weniger eindeutig werden bzw. sogar völlig an Bedeutung verlieren. Am Assayprinzip des erfindungsgemäßen Rezeptorbindungsassays sowie an der Tatsache, daß auch solche Assays in den Schutzbereich der vorliegenden Erfindung fallen, ändert das nichts.

Dadurch, daß das erfindungsgemäße Verfahren eine Immobilisierung des in der Meßlösung gebildeten TSH-Rezeptor-Komplexes mit einer direkt daran gebundenen Markierung oder einer nach der Immobilisierung eingeführten Markierung ermöglicht, und daß ferner Reagenzien mit anderen Markierungen als Radionukliden verwendet werden können, kann die praktische Handhabung von TSH-Rezeptor-Autoantikörper-Bestimmungsverfahren sehr viel besser als bisher möglich an Anforderungen der klinischen Praxis angepaßt werden. Auch die Inkubationsschemata können variiert werden, und das in den nachfolgenden Beispielen beschriebene Inkubationsschema erscheint nicht mehr zwingend erforderlich.

Als Festphasen können Kunststoffoberflächen, Mikropartikel, Magnetpartikel, Filter, Polymergelmaterialien und andere bekannte Festphasenträger eingesetzt werden. Die Möglichkeit, mit markierten Autoantikörpern und immobilisiertem TSH (bezüglich der Bindungsfähigkeit von TSH-Rezeptoren an immobilisiertes TSH vgl. Leedman, P.J. et al., J. Clin. Endocrinol. Metab. 69; S. 134-141, 1989) zu arbeiten, wird die Anwendungsbreite des Assays insbesondere auch im Hinblick auf eine beliebige Wahl geeigneter direkter oder indirekter Markierungen erheblich erhöht. Durch das erfindungsgemäße Verfahren werden TSH-Rezeptor-Autoantikörperassays auch automatisierbar. So kann das Assaydesign so angepaßt werden, daß es an bekannten automatisierten Systemen durchführbar ist (vgl. z.B. Elecsys-System der Firma Boehringer Mannheim oder ACS 180-System von Ciba Corning). Bei derartigen automatisierten Systemen wird im Rahmen einer automatischen Abarbeitung der Proben pipettiert, dann können Magnetpartikel, die mit einem geeigneten spezifischen Antikörper gegen den TSH-Rezeptor oder mit TSH beschichtet sind, in Vormischung mit markiertem TSH oder einem beliebig markierten Antikörper (z.B. Rutheniumkomplex- bzw. Acridiniumester-markiert) zugemischt werden, und abschließend erfolgt die Zugabe eines prinzipiell beliebigen geeigneten TSH-Rezeptor-Materials. Nach der Inkubation ist die übliche Fest-Flüssigtrennung möglich, und anschließend kann das Signal, gegebenenfalls nach Signalauslösung durch Zugabe geeigneter Reagenzien, bestimmt werden. Die beispielhaft angegebene Pipettierschrittfolge ist dabei variabel.

Nachfolgend wird das erfindungsgemäße. Verfahren anhand eines konkreten Ausführungsbeispiels unter Bezugnahme auf zwei Figuren noch näher erläutert.

Dabei zeigen:
- Figur 1: eine auf übliche Weise erhaltene Standardkurve für die im nachfolgenden Ausführungsbeispiel beschriebene Ausführungsform des erfindungsgemäßen Verfahrens; und
- Figur 2: eine Gegenüberstellung der mit dem erfindungsgemäßen Verfahren und dem herkömmlichen Radiorezeptorassay (TRAK-Assay® der Firma B.R.A.H.M.S Diagnostica GmbH) erhaltenen Meßergebnisse bei der Messung eines Kollektivs von Morbus Basedow-Patienten.

### Versuchsbeschreibung

### Materialien:

Im Rahmen der nachfolgend beschriebenen Versuche wurden verschiedene kommerziell erhältliche Materialien verwendet, und zwar insbesondere Komponenten des TRAK-Assay® der Firma B.R.A.H.M.S Diagnostica GmbH, eine NAP25-Entsalzungssäule der Firma Pharmacia zur Aufreinigung des für die Immobilisierung vorbereiteten Antikörpers, Carbolink®-Material der Firma Pierce als Festphasen-Trägermaterial sowie Natriumperjodat der Firma Fluka.

### 1. Herstellung von monoklonalen Antikörpern gegen den TSH-Rezeptor

Es wurden verschiedene monoklonale Antikörper gegen ausgewählte Peptide des hTSH-Rezeptors nach Verfahren des Standes der Technik hergestellt (s.o.). Für die weiteren Untersuchungen wurde ein monoklonaler Antikörper verwendet, der gegen ein Peptid gerichtet ist, das den Aminosäuren 20 bis 39 des vollständigen humanen TSH-Rezeptors (Aminosäuresequenz GGMGCSSPBCECHQEEDFRV) gerichtet ist.

### 2. Herstellung von festphasengebundenen monoklonalen Antikörpern

250 mg Perjodat werden in 20 ml einer 1:10 mit PBS (phosphatgepufferte Salzlösung; 50 mM Natriumphosphat, 100 mM NaCl, pH 7,5) verdünnten Asciteslösung, die den oben genannten monoklonalen Antikörper enthält, gelöst und für 30 min bei Raumtemperatur inkubiert. Die Reaktionslösung wird nach Vorschrift des Herstellers über eine NAP25-Entsalzungssäule (Pharmacia) entsalzt. Das entsalzte Protein wird mit in PBS gewaschenem Carbolink®-Material vermischt (10 ml).

Nach einer Inkubation über Nacht unter ständigem Umschütteln bei 4°C wurden 0,5 ml des erhaltenen Gels mit dem daran gebundenen monoklonalen Antikörper in Kunststoffsäulen (0,5 x 4 cm) gefüllt und mit 25 mM Natriumcitratpuffer, pH 2,5 gewaschen (Waschvolumen 2 ml). Die Säulen wurden in PBS (2 ml) äquilibriert und dann für die weiteren Untersuchungen verwendet.

### 3. Mit Hilfe der beschriebenen Säulen wurde unter Verwendung von Komponenten des TRAK-Assays® (B.R.A.H.M.S Diagnostica GmbH) ein Radiorezeptorassay durchgeführt

Die Durchführung des Radiorezeptorassays (Erstellung der Standardkurven, Messung von Patientenproben) erfolgte entsprechend den Vorschriften für die Durchführung des TRAK-Assays®. Dabei wurde folgendes Pipettierschema angewandt:
Es werden 50 µl der Probe pipettiert.
Anschließend werden 50 µl solubilisierter porciner TSH-Rezeptor dazupipettiert.
Es wird 15 min bei Raumtemperatur inkubiert.
Es werden 100 µl Tracer (radiojodiertes TSH) zupipettiert.
Der erhaltene Reaktionsansatz wird auf die mit dem monoklonalen Antikörper wie oben beladene Festphase (Säule mit Carbolink®-Material) überführt.
Der Reaktionsansatz wird eine Stunde bei Raumtemperatur inkubiert.
Die Säule wird mit 2 ml PBS gewaschen.
Die an die Festphase gebundene Radioaktivität wird in einem Gammacounter vermessen, und zwar entweder unter direkter Verwendung der Säule mit Festphase oder nach Eluierung der gebundenen Proteine mit 1 ml 25 mM Natriumcitratpuffer, pH 2,5.
Zur Erstellung einer Standardkurve für die Auswertung der Meßergebnisse von Patientenseren wurde entsprechend verfahren, wobei die Standards aus dem kommerziellen TRAK-Assay®, erweitert um einen Standard, der 2000 U TSH/ml enthält, verwendet wurden.

### Ergebnisse:

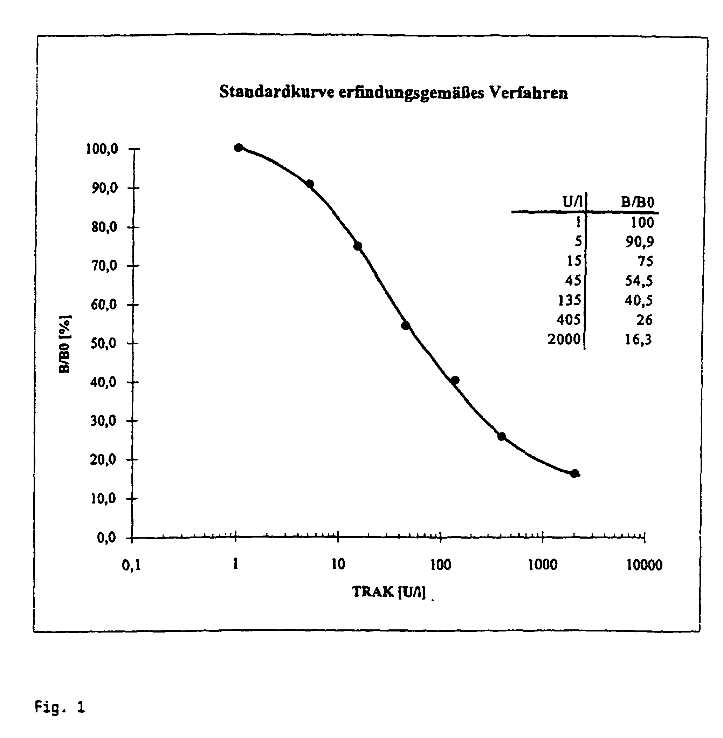

Unter Verwendung der Standards wurde eine Standardkurve (Fig. 1) erhalten, die der eines üblichen TRAK-Assays® entsprach, d.h. steigende Konzentrationen an zugesetztem unmarkiertem TSH führten zu einer Verringerung der gebundenen Radioaktivität.

Bei der Vermessung von Serumproben von Morbus Basedow-Patienten im kommerziellen TRAK-Assay® und nach dem erfindungsgemäßen Verfahren wurden folgende Ergebnisse erhalten:

Es wurden Seren von 45 gesunden Kontrollpersonen und von 39 Morbus Basedow-Patienten mit dem herkömmlichen TRAK-Assay® nach dem erfindungsgemäßen Verfahren vermessen. Die Meßergebnisse sind in Tabelle 1 zusammengefaßt.

Die Seren der Morbus Basedow-Patienten entstammen Proben vor der ersten Therapie oder innerhalb von sechs Wochen nach Beginn therapeutischer Maßnahmen.

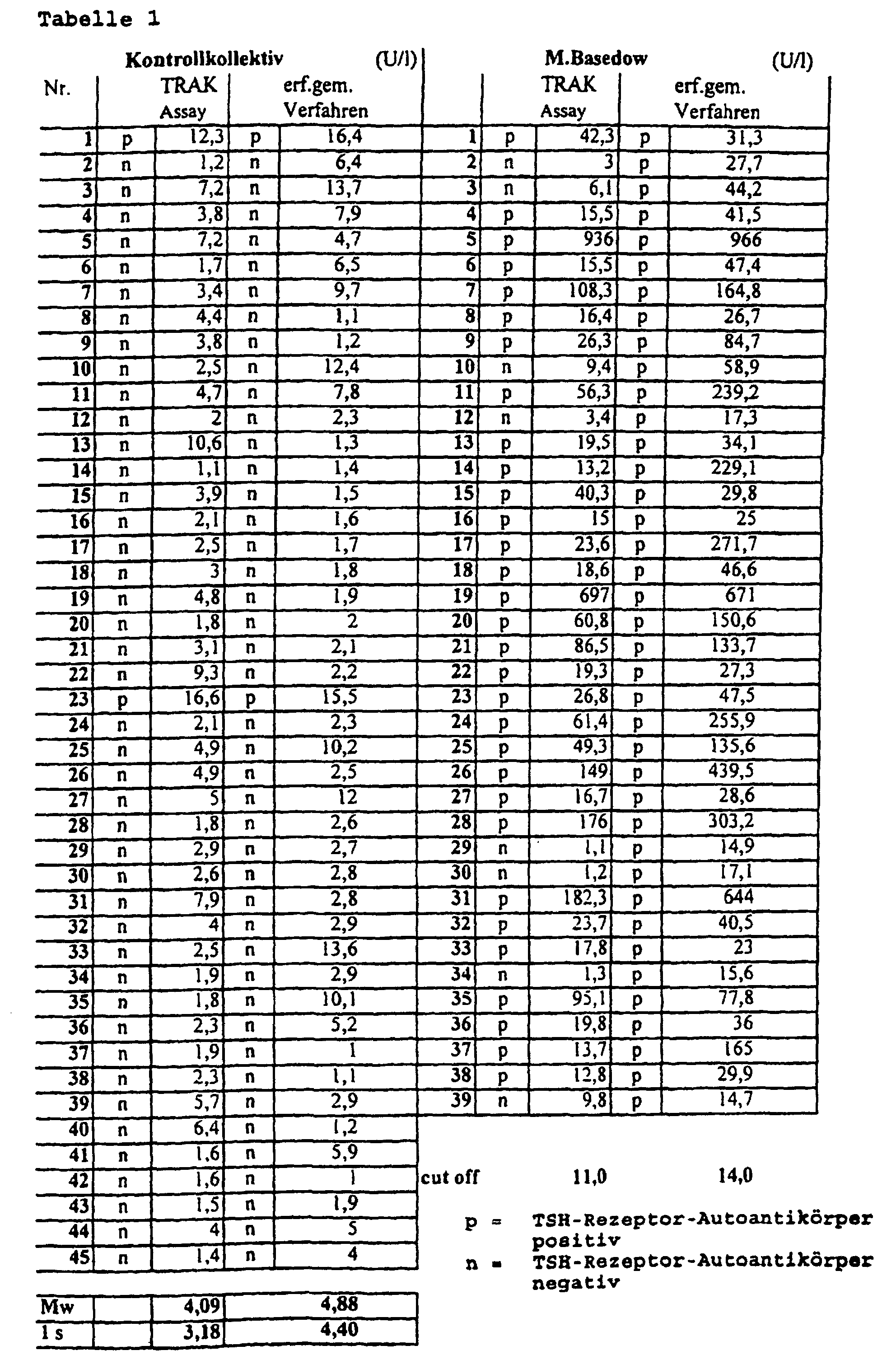

Der Mittelwert für die Vermessung der Autoantikörper-freien Seren der Kontrollgruppe (n-45) liegt bei 4,1 ± 6,4 (2s) U/ml für den TRAK-Assay® und bei 4,8 ± 8,8 (2s) U/ml bei dem erfindungsgemäßen Verfahren.

Um bei beiden Tests eine vergleichbare Spezifität von 95,5% einzuhalten, wurde der "Cut-Off" (Grenzlinie zwischen Werten für negative und positive Proben) für das herkömmliche Verfahren auf 11 U/ml und für das erfindungsgemäße Verfahren auf 14 U/ml festgelegt. Unter Anwendung dieser Cut-Off-Werte wurden im etablierten TRAK-Assay®-Radiorezeptor-Verfahren 79,5% der Basedow-Patienten (31 aus 39) als positiv erfaßt.

Eine Vermessung des gleichen Patientenkollektivs nach dem erfindungsgemäßen Verfahren ergab überraschend, daß 100% der Basedow-Patienten (39 aus 39) Autoantikörper-positiv gemessen wurden. Die im herkömmlichen TRAK-Assay® als negativ ermittelten Proben (Proben Nr. 2, 3, 12, 29, 30, 34, 39) wurden nach dem erfindungsgemäßen Verfahren eindeutig als positiv erfaßt.

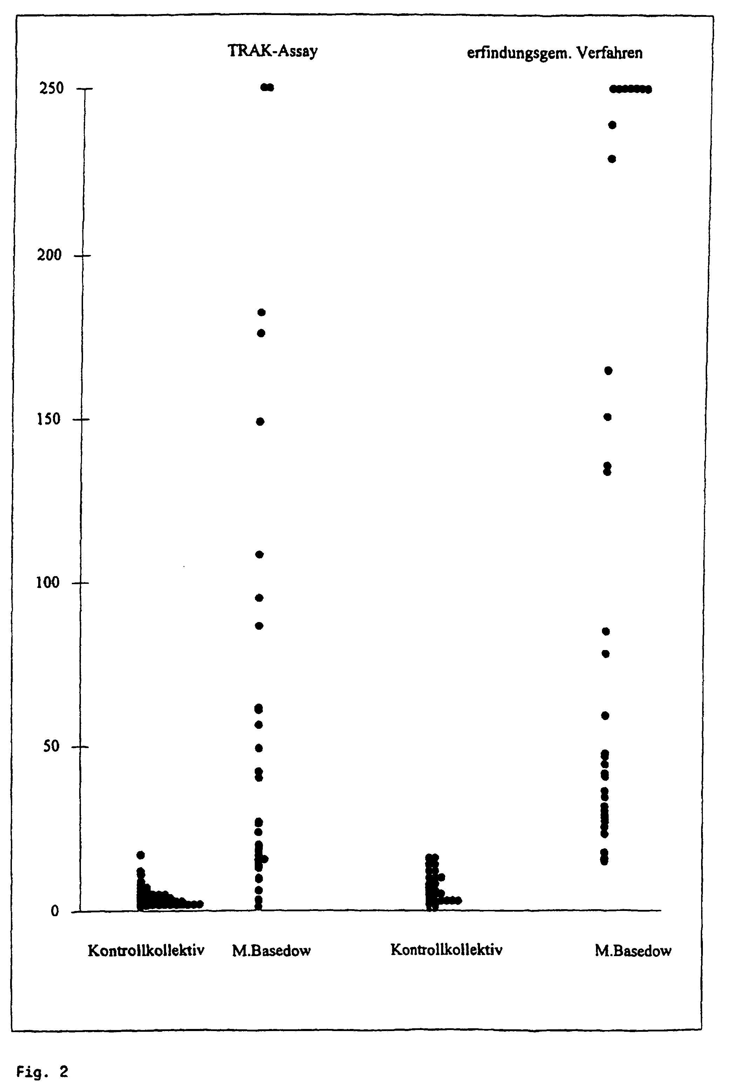

Darüberhinaus zeigte sich als weiterer Vorteil des erfindungsgemäßen Verfahrens, daß der überwiegende Teil der Proben mit signifikant stärkerem Meßsignal gefunden wurde. Die erhaltenen Ergebnisse sind zur Verdeutlichung in Fig. 2 graphisch dargestellt.

Durch die bei dem erfindungsgemäßen Verfahren bestehende zusätzliche Möglichkeit der Störung der. Wechselwirkung zwischen festphasengebundenem spezifischen Antikörper und dem TSH-Rezeptor-Komplex mit daran gebundenem radioaktiv markiertem TSH durch in der Probe vorhandene Autoantikörper wird nicht nur die klinische Wertigkeit des Assays für die Morbus Basedow-Diagnostik erhöht, sondern zusätzlich wird auch bei in beiden Tests positiv identifizierten Patienten nach dem erfindungsgemäßen Verfahren ein wesentlich stärkeres und damit sichereres Meßsignal erzeugt. Die klinische Wertigkeit der Bestimmung von TSH-Rezeptor-Autoantikörpern, deren Auftreten für einen Morbus Basedow charakteristisch ist, wird somit entscheidend erhöht.

## Patentansprüche

1. Kompetitiver Rezeptorbindungsässay zur Bestimmung von TSH-Rezeptor-Autoantikörpern in einer biologischen Probe, bei dem man die Probe in einer Reaktionsmischung gleichzeitig oder nacheinander mit (i) einem TSH-Rezeptor in Form einer solubilisierten nativen humanen oder tierischen oder rekombinanten TSH-Rezeptor-Präparation, (ii) einem primären Kompetitor für wenigstens einen Teil der in der Probe zu erwartenden TSH-Rezeptor-Autoantikörper und (iii) einem Mittel für die Abtrennung eines Komplexes aus dem TSH-Rezeptor und daran gebundenen Bestandteilen der. Reaktionsmischung von der flüssigen Phase umsetzt, und bei dem man die Anwesenheit und/oder Menge der zu bestimmenden TSH-Rezeptor-Autoantikörper in der biologischen Probe auf der Basis der Menge eines Kompetitors in dem von der flüssigen Phase abgetrennten TSH-Rezeptor-Komplex oder anhand der Restmenge des ungebundenen primären Kompetitors in der Flüssigphase ermittelt, wozu man den Kompetitor in markierter Form einsetzt oder nach der Fest-Flüssig-Trennung markiert,
**dadurch gekennzeichnet, daß** man die Umsetzung ferner in Gegenwart mindestens eines monoklonalen oder polyklonalen Antikörpers durchführt, der spezifisch gegen eine Peptidsequenz des TSH-Rezeptors gerichtet ist, die ausgewählt ist aus Peptiden mit den Aminosäuren 20-39, 32-54, 287-301, 361-381 oder 739-758 der Aminosäuresequenz des vollständigen TSH-Rezeptors, und daß dieser spezifische Antikörper als Mittel zur Immobilisierung eines Komplexes aus TSH-Rezeptor und primärem Kompetitor an eine Festphase gebunden ist, ohne dass die Bindungsfähigkeit des TSH-Rezeptors gegen zur Inhibierung des TSH-Bindung befähigte Autoantikörper (TBII-Autoantikörper) und gegen markiertes TSH nennenswert beeinträchtigt ist, und/oder als sekundärer Kompetitor für einen weiteren Teil der in einer Probe zu erwartenden TSH-Rezeptor-Autoantikörper eingesetzt wird, wobei der primäre oder der sekundäre Kompetitor markiert oder selektiv markierbar ist.

2. Rezeptorbindungsassay nach Anspruch 1, **dadurch gekennzeichnet, daß** der mindestens eine spezifische Antikörper in festphasengebundener Form eingesetzt wird und der primäre Kompetitor markiertes TSH oder ein markierter weiterer TSH-Rezeptor-Antikörper ist, wobei der festphasengebundene spezifische Antikörper unbeeinflußt von der Bindung des primären Kompetitors an den TSH-Rezeptor bindet.

3. Rezeptorbindungsassay nach Anspruch 2, **dadurch gekennzeichnet, daß** der festphasengebunde Antikörper gleichzeitig ein sekundärer Kompetitor ist, der mit solchen TSH-Rezeptor-Autoantikörpern aus der Probe, die die Bindung des primären . Kompetitors an den TSH-Rezeptor nicht behindern, um ein von dem primären Kompetitor nicht erkanntes Epitop kompetiert.

4. Rezeptorbindungsassay nach Anspruch 2, **dadurch gekennzeichnet, daß** der markierte weitere TSH-Rezeptor-Antikörper mit TSH um Epitope des TSH-Rezeptors kompetiert.

5. Rezeptorbindungsassay nach Anspruch 3, **dadurch gekennzeichnet, daß** der festphasengebundene Antikörper als sekundärer Kompetitor mit solchen TSH-Rezeptor-Autoantikörpern aus der Probe kompetiert, deren Auftreten für den Morbus Basedow charakteristisch ist, die jedoch nicht mit TSH kompetieren.

6. Rezeptorbindungsassay nach Anspruch 5, **dadurch gekennzeichnet, daß** die mit dem sekundären Kompetitor kompetierenden TSH-Rezeptor-Autoantikörper zu den stimulierenden Autoantikörpern (TSI) gehören.

7. Rezeptorbindungsassay nach Anspruch 1, **dadurch gekennzeichnet, daß** der mindestens eine Antikörper in markierter Form als sekundärer Kompetitor in der flüssigen Phase eingesetzt wird und daß ein festphasengebundenes TSH als Mittel für die Abtrennung eines Komplexes aus dem TSH-Rezeptor und daran gebundenen Bestandteilen der Reäktionsmischung sowie als primärer Kompetitor eingesetzt wird.

8. Rezeptorbindungsassay nach einem der Ansprüche 1 bis 7, **dadurch gekennzeichnet, daß** der mindestens eine weitere Antikörper durch an sich bekannte Immunisierung eines geeigneten Tiers mit einem rekombinanten partiellen TSH-Rezeptor, insbesondere der extrazellulären Domäne oder einer extrazellulären Schlinge der membranverankerten Domäne eines TSH-Rezeptors, und durch affinitätschromatographische Fraktionierung der gebildeten Antikörper unter Verwendung immobilisierter Teilpeptide des zur Immnunisierung verwendeten partiellen TSH-Rezeptors hergestellt wurde.

9. Rezeptorbindungsassay nach einem der Ansprüche 1 bis 7, **dadurch gekennzeichnet, daß** der mindestens eine weitere Antikörper ein durch an sich bekannte Immunisierung eines geeigneten Tiers mit einem rekombinanten partiellen TSH-Rezeptor, insbesondere dem rekombinanten extrazellulären Teil eines TSH-Rezeptors oder einem kürzeren Teilpeptid davon, an sich bekannte Fusion antikörperproduzierender Milzzellen mit Myelomzellen und an sich bekannte Selektion einzelner antikörperproduzierender Hybridzellen und deren Kultivierung hergestellter monoklonaler Antikörper ist.

10. Rezeptorbindungsassay nach einem der Ansprüche 1 bis 9, **dadurch gekennzeichnet, daß** man als Festphase Träger in Form von Partikeln, Teströhrchen oder Mikrotiterplatten aus Kunststoff oder Glas oder in Form von magnetischen Polymerpartikeln, Polymergelen oder Filtern verwendet und man den mindestens einen Antikörper oder TSH an einen solchen Träger direkt oder indirekt gebunden einsetzt.

11. Rezeptorbindungsassay nach einem der Ansprüche 1 bis 10, **dadurch gekennzeichnet, daß** man zur Markierung des primären oder sekundären Kompetitors eine an sich bekannte Markierung verwendet, die ausgewählt ist aus einem Radionuklid, einem Enzym, einem Enzymsubstrat oder einem Bestandteil eines Chemilumineszenz- oder Fluoreszenz-Markierungssystems.

12. Rezeptorbindungsassay nach einem der Ansprüche 1 bis 10, **dadurch gekennzeichnet, daß** man einen primären oder sekundären Kompetitor einsetzt, der an eine Komponente eines spezifischen Bindungssystems gebunden ist, und daß man den gebildeten TSH-Rezeptor-Komplex durch Umsetzung mit einem Reaktionspartner markiert oder immobilisiert, der die zweite Komponente des spezifischen Bindungssystems aufweist, die an eine detektierbare Markierung oder eine Festphase gebunden ist.

13. Rezeptorbindungsassay nach einem der Ansprüche 1 bis 12, **dadurch gekennzeichnet, daß** die zu bestimmenden TSH-Rezeptor-Autoantikörper rezeptorstimulierende Autoantikörper sind, deren Auftreten in einem Humanserum für den Morbus Basedow charakteristisch ist.

14. Reagenziensatz zur Durchführung eines kompetitiven Rezeptorbindungsassays nach einem der Ansprüche 1 bis 13, **dadurch gekennzeichnet, daß** er, neben weiteren üblichen Komponenten eines derartigen Reagenziensatzes, enthält:
(i) eine solubilisierte TSH-Rezeptor-Präparation,
(ii) markiertes TSH oder einen markierten spezifischen TSH-Rezeptor-Antikörper, und
(iii) einen Festphasenträger, an den ein spezifischer monoklonaler oder polyklonaler Antikörper gebunden ist, der spezifisch gegen eine Peptidsequenz des TSH-Rezeptors gerichtet ist, die ausgewählt ist aus Peptiden mit den Aminosäuren 20-39, 32-54, 287-301, 361-381 oder 739-758 der Aminosäuresequenz des vollständigen TSH-Rezeptors.

## Claims

1. Competitive receptor binding assay for the determination of TSH receptor autoantibodies in a biological sample, in which the sample in a reaction mixture is reacted simultaneously or in succession with (i) a TSH receptor in the form of a solubilized native human or animal or recombinant TSH receptor preparation, (ii) a primary competitor for at least a part of the TSH receptor autoantibodies to be expected in the sample and (iii) an agent for separating a complex of the TSH receptor and components of the reaction mixture which are bound thereto from the liquid phase, and in which the presence and/or amount of the TSH receptor autoantibodies to be determined in the biological sample are determined on the basis of the amount of a competitor in the TSH receptor complex separated from the liquid phase or on the basis of the residual amount of the unbound primary competitor in the liquid phase, for which purpose the competitor is used in labelled form or is labelled after the solid-liquid separation,
**characterized in that** the reaction is furthermore carried out in the presence of a monoclonal or polyclonal antibody which is directed specifically against a peptide sequence of the TSH receptor, which is selected from peptides with the amino acids 20-39, 32-54, 287-301, 361-381 or 739-758 of the amino acid sequence of the complete TSH receptor, and that this specific antibody is bound to a solid phase as an agent for immobilizing a complex of TSH receptor and primary competitor, without the binding capability of the TSH receptor with respect to autoantibodies capable of inhibiting TSH binding (TBII autoantibodies) and with respect to labelled TSH being significantly impaired, and/or as a secondary competitor for a further part of the TSH receptor autoantibodies to be expected in a sample, the primary or the secondary competitor being labelled or being capable of being selectively labelled.

2. Receptor binding assay according to Claim 1, **characterized in that** the at least one specific antibody is used in a form bound to a solid phase and the primary competitor is labelled TSH or a labelled further TSH receptor antibody, the specific antibody bound to the solid phase binding to the TSH receptor, uninfluenced by the binding of the primary competitor.

3. Receptor binding assay according to Claim 2, **characterized in that** the antibody bound to the solid phase is simultaneously a secondary competitor which competes with such TSH receptor autoantibodies from the sample which do not hinder the binding of the primary competitor to the TSH receptor, for an epitope not recognized by the primary competitor.

4. Receptor binding assay according to Claim 2, **characterized in that** the labelled further TSH receptor antibody competes with TSH for epitopes of the TSH receptor.

5. Receptor binding assay according to Claim 3, **characterized in that** the antibody bound to the solid phase competes as a secondary competitor with such TSH receptor autoantibodies from the sample whose occurrence is characteristic for Graves' disease but which do not compete with TSH.

6. Receptor binding assay according to Claim 5, **characterized in that** the TSH receptor autoantibodies competing with the secondary competitor belong to the stimulating autoantibodies (TSI).

7. Receptor binding assay according to Claim 1, **characterized in that** the at least one antibody is used in labelled form as a secondary competitor in the liquid phase and that TSH bound to the solid phase is used as agent for separating a complex of the TSH receptor and components of the reaction mixture which are bound thereto, and as a primary competitor.

8. Receptor binding assay according to any of Claims 1 to 7, **characterized in that** the at least one further antibody was prepared by immunization, known per se, of a suitable animal with a recombinant partial TSH receptor, in particular the extracellular domain or an extracellular loop of the membrane-anchored domain of a TSH receptor, and by fractionation of the resulting antibodies by affinity chromatography with the use of immobilized partial peptides of the partial TSH receptor used for the immunization.

9. Receptor binding assay according to any of Claims 1 to 7, **characterized in that** the at least one further antibody is a monoclonal antibody prepared by immunization, known per se, of a suitable animal with a recombinant partial TSH receptor, in particular the recombinant extracellular part of a TSH receptor or a shorter partial peptide thereof, fusion, known per se, of antibody-producing splenocytes with myelocytes and selection, known per se, of individual antibody-producing hybrid cells and the cultivation thereof.

10. Receptor binding assay according to either of Claims 1 to 9, **characterized in that** substrates in the form of particles, test tubes or microtitre plates of plastic or glass or in the form of magnetic polymer particles, polymer gels or filters are used as the solid phase and the at least one antibody or TSH is used in a form bound directly or indirectly to such a substrate.

11. Receptor binding assay according to any of Claims 1 to 10, **characterized in that** a tracer which is known per se and which is selected from a radionucleid, an enzyme, an enzyme substrate or a component of a chemiluminesent or fluorescent labelling system is used for labelling the primary or secondary competitor.

12. Receptor binding assay according to any of Claims 1 to 10, **characterized in that** a primary or secondary competitor which is bound to a component of a specific binding system is used and that the TSH receptor complex formed is labelled or immobilized by reaction with a reactant which has the second component of the specific binding system, which second component is bound to a detectable tracer or a solid phase.

13. Receptor binding assay according to any of Claims 1 to 12, **characterized in that** the TSH receptor autoantibodies to be determined are receptor-stimulating autoantibodies, the occurrence of which in a human serum is characteristic of Graves' disease.

14. Reagent kit for carrying out a competitive receptor binding assay according to any of Claims 1 to 13, **characterized in that** it contains, in addition to further conventional components of such a reagent kit:
(i) a solubilized TSH receptor preparation,
(ii) labelled TSH or a labelled specific TSH receptor antibody and
(iii) a solid-phase substrate to which a specific monoclonal or polyclonal antibody is bound, which is directed specifically against a peptide sequence of the TSH receptor, which is selected from peptides with the amino acids 20-39, 32-54, 287-301, 361-381 or 739-758 of the amino acid sequence of the complete TSH receptor.

## Revendications

1. Détermination de la liaison compétitive à un récepteur, pour la détermination d'auto-anticorps du récepteur de TSH dans un échantillon biologique, dans laquelle, dans un mélange de réaction, on fait réagir l'échantillon simultanément ou successivement avec (i) un récepteur de TSH qui présente la forme d'une préparation de récepteur de TSH natif, humain, animal ou recombiné, solubilisé, (ii) un compétiteur primaire pour au moins une partie des auto-anticorps d'un récepteur de TSH que l'on s'attend à trouver dans l'échantillon et (iii) un moyen pour séparer de la phase liquide un complexe constitué du récepteur de TSH et des composants du mélange de réaction qui y sont liés, et dans laquelle on détermine la présence et/ou la quantité des auto-anticorps du récepteur de TSH qu'il faut déterminer dans l'échantillon biologique à partir de la quantité d'un compétiteur présent dans le complexe de récepteur de TSH séparé de la phase liquide ou à l'aide de la quantité résiduelle de compétiteur primaire qui n'a pas réagi et qui est présente dans la phase liquide, le compétiteur étant utilisé dans ce but sous forme marquée ou étant marqué après la séparation solide-liquide,
**caractérisée en ce que** l'on réalise en outre la réaction en présence d'au moins un anticorps monoclonal ou polyclonal qui est dirigé spécifiquement contre une séquence de peptides du récepteur de TSH qui est sélectionnée parmi les peptides qui comptent les acides aminés 20 à 39, 32 à 54, 287 à 301, 361 à 381 ou 739 à 758 de la séquence complète des acides aminés du récepteur de TSH et **en ce que** cet anticorps spécifique qui sert de moyen d'immobilisation d'un complexe constitué du récepteur de TSH et du compétiteur primaire est lié à une phase solide, sans que la capacité de liaison du récepteur de TSH à l'auto-anticorps capable d'inhiber la liaison au TSH (auto-anticorps TBII) et à la TSH marquée soit significativement dégradée, et/ou est utilisé comme compétiteur secondaire pour une autre partie des auto-anticorps du récepteur de TSH que l'on s'attend à trouver dans un échantillon, le compétiteur primaire ou le compétiteur secondaire étant marqués ou pouvant être marqués sélectivement.

2. Détermination de la liaison à un récepteur selon la revendication 1, **caractérisée en ce que** le ou les auto-anticorps spécifiques présents sont utilisés sous forme liée à une phase solide, le compétiteur primaire étant une TSH marquée ou un autre anticorps marqué du récepteur de TSH, l'auto-anticorps spécifique lié à la phase solide se liant au récepteur de TSH sans être influencé par la liaison du compétiteur primaire.

3. Détermination de la liaison à un récepteur selon la revendication 2, **caractérisée en ce que** l'anticorps lié à la phase solide est en même temps un compétiteur secondaire qui entre en compétition vis-à-vis d'un épitope non reconnu par le compétiteur primaire avec les auto-anticorps du récepteur de TSH qui n'empêchent pas la liaison du compétiteur primaire avec le récepteur de TSH.

4. Détermination de la liaison à un récepteur selon la revendication 2, **caractérisée en ce que** l'autre anticorps marqué du récepteur de TSH entre en compétition avec la TSH vis-à-vis de l'épitope du récepteur de TSH.

5. Détermination de la liaison à un récepteur selon la revendication 3, **caractérisée en ce que** l'anticorps lié à une phase solide entre en compétition en tant que compétiteur secondaire avec les auto-anticorps du récepteur de TSH qui proviennent de l'échantillon et dont la présence est caractéristique de la maladie de Basedow mais qui n'entrent pas en compétition avec la TSH.

6. Détermination de la liaison à un récepteur selon la revendication 5, **caractérisée en ce que** les auto-anticorps du récepteur de TSH qui entrent en compétition avec le compétiteur secondaire appartiennent à la classe des auto-anticorps stimulants (TSI).

7. Détermination de la liaison à un récepteur selon la revendication 1, **caractérisée en ce que** le ou les anticorps présents sont utilisés sous forme marquée comme compétiteurs secondaires dans la phase liquide et **en ce qu'**une TSH liée à la phase solide est utilisée comme moyen de séparation d'un complexe constitué du récepteur de TSH et des composants du mélange de réaction qui y sont liés ainsi que comme compétiteur primaire.

8. Détermination de la liaison à un récepteur selon l'une des revendications 1 à 7, **caractérisée en ce que** le ou les anticorps présents sont préparés par immunisation connue en soi d'un animal approprié par un récepteur de TSH partiellement recombiné, en particulier du domaine extracellulaire ou d'une boucle extracellulaire du domaine ancré à la membrane d'un récepteur de TSH, et par fractionnement par chromatographie à affinité des anticorps ainsi formés, avec recours à des peptides partiels immobilisés du récepteur partiel de TSH utilisé pour l'immunisation.

9. Détermination de la liaison à un récepteur selon l'une des revendications 1 à 7, **caractérisée en ce que** le ou les autres anticorps présents est un anticorps monoclonal préparé par immunisation connue en soi d'un animal approprié par un récepteur partiel recombiné de TSH, en particulier la partie extracellulaire recombinée d'un récepteur de TSH ou un peptide partiel plus court de ce dernier, par fusion connue en soi de cellules de rate productrices d'anticorps avec des cellules de myélome et sélection connue en soi de cellules hybrides individuelles qui produisent des anticorps, ainsi que leur culture.

10. Détermination de la liaison à un récepteur selon l'une des revendications 1 à 9, **caractérisée en ce que** comme phase solide, on utilise des supports qui présentent la forme de particules, de tubes à essai ou de plaques de microtitration en matière synthétique ou en verre, ou sous la forme de particules polymères magnétiques, de gels polymères magnétiques ou de filtres magnétiques et **en ce que** l'on utilise le ou les anticorps présents ou la TSH liés directement ou indirectement à l'un de ces supports.

11. Détermination de la liaison à un récepteur selon l'une des revendications 1 à 10, **caractérisée en ce que** pour le marquage du compétiteur primaire ou du compétiteur secondaire, on utilise un marqueur connu en soi qui est sélectionné parmi un radionucléide, une enzyme, un substrat d'enzyme ou un composant d'un système de marquage par chimioluminescence ou fluorescence.

12. Détermination de la liaison à un récepteur selon l'une des revendications 1 à 10, **caractérisée en ce que** l'on utilise un compétiteur primaire ou un compétiteur secondaire est qui lié à un composant d'un système de liaison spécifique et **en ce que** l'on marque ou immobilise le complexe ainsi formé avec le récepteur de TSH par réaction avec un partenaire de réaction qui présente le deuxième composant du système de marquage spécifique qui est lié à un marqueur détectable ou à une phase solide.

13. Détermination de la liaison à un récepteur selon l'une des revendications 1 à 12, **caractérisée en ce que** les auto-anticorps du récepteur de TSH à déterminer sont des auto-anticorps qui stimulent le récepteur et dont la présence dans un sérum humain est caractéristique de la maladie de Basedow.

14. Trousse de réactifs en vue de la mise en oeuvre d'une détermination de la liaison à un récepteur selon l'une des revendications 1 à 13, **caractérisée en ce qu'**en plus d'autres composants habituels d'une telle trousse de réactifs, elle contient :
(i) une préparation solubilisée de récepteur de TSH,
(ii) de la TSH marquée ou un anticorps spécifique marqué du récepteur de TSH et
(iii) un support en phase solide auquel est lié un anticorps monoclonal ou polyclonal spécifique qui est dirigé spécifiquement contre une séquence de peptides du récepteur de TSH qui est sélectionnée parmi les peptides qui comptent les acides aminés 20 à 39, 32 à 54, 287 à 301, 361 à 381 ou 739 à 758 de la séquence complète des acides aminés du récepteur de TSH.
